# EUROPEAN PATENT SPECIFICATION

(11) **EP 3 100 721 B1**
(45) Date of publication and mention of the grant of the patent: **26.08.2020**
(21) Application number: 15742744.4
(22) Date of filing: 22.01.2015
(51) Int. Cl.: A61K 9/66, A61K 9/48, A61K 47/04, A61K 47/44, A61K 31/714

(54) **CAPSULE FORMULATION**
KAPSELFORMULIERUNG
FORMULATION DE CAPSULE

(30) Priority: 31.01.2014 JP 2014017685
(43) Date of publication of application: 07.12.2016
(73) Proprietor: Fuji Capsule Co., Ltd., Fujinomiya-shi, Shizuoka 418-0112 (JP)
(72) Inventor: NAKANO, Hiroshi, Fujinomiya-shi Shizuoka 418-0112 (JP); NISHIMURA, Naoki, Fujinomiya-shi Shizuoka 418-0112 (JP); MASUDA, Koji, Fujinomiya-shi Shizuoka 418-0112 (JP); SHIMOKAWA, Yoshiyuki, Fujinomiya-shi Shizuoka 418-0112 (JP); KATO, Kenji, Fujinomiya-shi Shizuoka 418-0112 (JP)
(74) Representative: Script IP Limited
(86) International application number: PCT/JP2015/000291
(87) International publication number: WO 2015/115072

(56) References cited:
- EP-A2- 0 212 875
- CN-A- 102 133 406
- JP-A- S5 480 407
- JP-A- H08 511 559
- JP-A- 2002 519 370
- JP-A- 2005 501 914
- US-A- 5 891 470

## Description

### Technical Field

The present invention relates to a capsule formulation, and more specifically to a capsule formulation, which is produced by encapsulating with a hydrophilic film, a capsule content formed by enclosing an aqueous solution in an oily substance containing a porous microparticulate powder.

### Background Art

Conventionally, various capsules for encapsulating functional foods, drugs, etc. therein have been reported. As film-forming materials used in these capsules, gelatin (see patent document 1 and patent document 2), a mixture of agar and a water-soluble polymer (see patent document 3), alginate (see patent document 4 and patent document 5), and the like have been used.

In general, water is used as a solvent for hydrophilic substances. When gelatin or a mixture of agar and a water-soluble polymer is used as a film-forming material, and a capsule formulation encapsulating a water-containing material is produced, the softening of the capsule film occurs due to the compatibility of a coating solution with water upon encapsulation or the transfer of water after encapsulation. Accordingly, a hydrophilic substance cannot be stably encapsulated in a capsule under the current circumstances.

In the case of a capsule produced using gelatin as a film-forming material, since the film is hydrophilic, the capsule is problematic in that the film is dissolved in water, if a material containing water, for example, a food extract such as a garlic extract, a coffee extract or an Aloe vera extract is encapsulated therein. Thus, it has been necessary to process a material to be encapsulated to the form of powders, or to suspend it in hardened oil before the material is encapsulated in a capsule.

Moreover, in the case of a capsule comprising a mixture of agar and a water-soluble polymer as a film-forming material, a film is formed by dissolving agar in water, and then adding a water-soluble polymer such as carrageenan to the obtained solution. Since the thus formed film also has hydrophilicity, the capsule is also problematic in that the film is dissolved in water if a water-containing material is encapsulated therein. Hence, this capsule has been used in a case where a capsule encapsulates a content that has been dissolved in oil and fat (see patent document 3).

Furthermore, as a method for producing a capsule using alginate as a film-forming material, a method, which comprises dispersing or dissolving a desired material in an aqueous solution of sodium alginate, then adding the obtained solution dropwise to a multivalent metal salt aqueous solution to form a water-insoluble film outside of the formed droplets, and then solidifying even insides of the droplets, so as to obtain a strong capsule, has been known, for example (see patent document 4). It is considered that this method requires complicated steps such as washing and/or drying during production of a capsule formulation.

Meanwhile, with regard to encapsulation of a hydrophilic substance, a method of encapsulating a hydrophilic substance by interposing a lower fatty acid ester of sucrose between the hydrophilic substance and a film has been proposed (see patent document 5). However, since a hydrophobic film consisting of such a lower fatty acid ester of sucrose is an aggregate of low-molecular-weight compounds, the aforementioned method is problematic in that a hydrophilic substance is not effectively blocked by the film.

Further, as a result of the technical innovation in recent years, a technique of purifying and/or extracting components from animals, plants and the like, and a technique of purifying components by chemical synthesis have been developed, and various components and extracts could have been produced. There are many capsule formulations formed from mixtures of such two or more components. However, such capsule formulations have been problematic in that incompatible components, being likely to cause negative interaction between them, cannot be filled in a single capsule.

In addition to the above described methods, a method of allowing a soft capsule film to comprise silicon dioxide microparticles has been proposed (see patent documents 6 and 7). However, this method has aimed to eliminate the adhesive property of the soft capsule.

D1 (US5891470) discloses a formulation (see D1 at column 2, lines 13-17; Example 4; and claims 13 and 21) comprising the following:
(a) a soft gelatin shell; and
(b) a fill material within the shell, the fill material comprising:
   (i) an emulsion comprising a polyethylene glycol, a silicone oil and up to 10% of water by weight of the emulsion; and
   (ii) retinol-impregnated porous microparticles.

D2 (EP0212875) discloses (see claim 1 of D2) a composition comprising lipid, non-lipid liquid (for example, water) and pharmaceutical ingredients, characterized by the fact that the ingredients are emulsified and encapsulated in a gelatin capsule. Claim 13 of D2 discloses adding fumed silica to the emulsion.

### Prior Art Documents

### Patent Documents

Patent Document 1: Japanese unexamined Patent Application Publication No. 59-139317
Patent Document 2: Japanese unexamined Patent Application Publication No. 61-151127
Patent Document 3: Japanese unexamined Patent Application Publication No. 9-25228
Patent Document 4: Japanese unexamined Patent Application Publication No. 3-68508
Patent Document 5: Japanese unexamined Patent Application Publication No. 3-52639
Patent Document 6: Japanese unexamined Patent Application Publication No. 6-247845
Patent Document 7: Japanese unexamined Patent Application Publication No. 8-34727

### Summary of the Invention

### Object to be Solved by the Invention

The present invention has been made under the aforementioned conventional circumstances. It is an object of the present invention to provide a capsule formulation capable of stably retaining a hydrophilic substance for a long period of time and also capable of comprising incompatible components in a single capsule.

### Means to Solve the Object

As a result of intensive studies directed towards achieving the aforementioned object, the present inventors have found that, when an aqueous solution, in which a hydrophilic substance has been dissolved, is added to a liquid mixture formed by adding a porous microparticulate powder to cooking oil to prepare a capsule content, and the capsule content is then encapsulated with a gelatin film so as to produce a capsule formulation, the softening of the gelatin film does not occur, and also, the hydrophilic substance can be stably retained, although a component whose mixing is prohibited is comprised in a single capsule, thereby completing the present invention.

Specifically, the present invention relates to: [1] a capsule formulation, produced by encapsulating with a hydrophilic film, a capsule content formed by enclosing soft microparticles, which are produced by dissolving a water-soluble polymer in water, in an oily substance containing a porous microparticulate powder; the invention also relates to the capsule formulation as described above which comprises 0.1 to 20 parts by weight of porous microparticulate powder based on 100 parts by weight of oily substance; the invention also relates to the capsule formulation as described above which is a soft capsule formulation; the invention also relates to the capsule formulation as described above wherein the porous microparticulate powder is a fumed silica.

### Effect of the Invention

According to the present invention, provided is a capsule formulation capable of stably retaining a hydrophilic substance in an aqueous solution over a long period of time and also capable of containing incompatible components, in a single capsule.

### Brief Description of Drawings

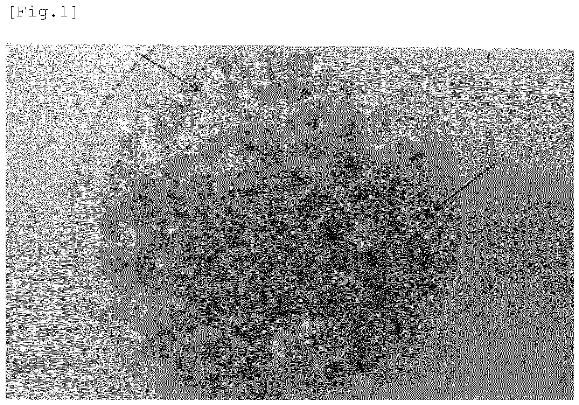

[Figure 1] Figure 1 is a photograph showing the agar granule-containing soft capsules obtained in Example 1.

### Mode of Carrying Out the Invention

The capsule formulation described herein is not particularly limited, as long as it is a capsule formulation produced by encapsulating with a hydrophilic film, a capsule content formed by enclosing an aqueous solution in an oily substance containing a porous microparticulate powder. The present capsule formation is preferably a soft capsule formulation.

In the present invention, examples of the porous microparticulate powder include: amorphous synthetic silica such as fumed silica or wet silica; and known microparticles such as the microparticles of alumina, alumina hydrate, calcium carbonate, magnesium carbonate, or titanium dioxide. From the viewpoint of achieving high porosity, amorphous synthetic silica such as fumed silica or wet silica, or alumina or alumina hydrate is preferable; amorphous synthetic silica such as fumed silica or wet silica is more preferable; and fumed silica is further preferable.

The amorphous synthetic silica is classified into fumed silica, wet silica, and other types of silica, depending on the production method thereof. In addition, the wet silica is further classified into precipitation process silica, gel process silica, and sol process silica, depending on the production method thereof.

The fumed silica is also referred to as dry process silica, in contrast to wet process silica, and the fumed silica is generally produced by a flame hydrolysis method. Specifically, a method for producing fumed silica by burning silicon tetrachloride together with hydrogen and oxygen has been generally known. Instead of silicon tetrachloride, silanes such as methyltrichlorosilane or trichlorosilane can also be used singly or in combination with silicon tetrachloride. As such fumed silica, AEROSIL (registered trademark) (manufactured by NIPPON AEROSIL CO., LTD.) and REOLOSIL (registered trademark) (manufactured by Tokuyama) are commercially available.

The precipitation process silica is produced by reacting silicate soda with sulfuric acid under alkaline conditions. This silica is produced through processes, such as agglutination and/or precipitation of silica particles, which have grown, and the subsequent processes such as filtration, water washing, drying, and crushing/classification. As such precipitation process silica, NIPSIL (registered trademark) (manufactured by TOSOH SILICA CORPORATION) is commercially available.

The gel process silica is produced by reacting silicate soda with sulfuric acid under acidic conditions. During maturation, microparticles are dissolved, and are re-precipitated such that they bind between other primary particles. Thus, clear primary particles disappear, and relatively hard aggregated particles having an internal void structure are formed. As such gel process silica, NIPGEL (registered trademark) (manufactured by TOSOH SILICA CORPORATION), and SYLOID and SYLOJET (manufactured by GRACE JAPAN CO., LTD.) are commercially available.

The sol process silica is also referred to as colloidal silica, and this silica is obtained by thermal maturation of silica sol, which is obtained by the metathesis of silicate soda with acid or the like, or through an ion exchange resin layer. As such sol process silica, SNOWTEX (registered trademark) (manufactured by NISSAN CHEMICAL INDUSTRIES, LTD.) is commercially available.

The specific surface area of a porous microparticulate powder is 100 m²/g or more, and is preferably 100 to 500 m²/g.

In the present invention, two or more types of a porous microparticulate powder can be used in combination. Examples of such combined use include the combined use of crushed fumed silica with wet silica, the combined use of finely crushed wet silica with alumina or alumina hydrate, and the combined use of fumed silica with alumina or alumina hydrate.

Examples of the oily substance used in the present invention include: animal and vegetable oils, such as safflower oil, linseed oil, sesame oil, olive oil, soybean oil, mustard oil, rapeseed oil, corn oil, castor oil, evening primrose oil, palm kernel oil, jojoba oil, cottonseed oil, palm oil, peanut oil, cacao oil, lard, EPA, DHA, shark liver oil, cod liver oil, hardened oil and partially hardened oil; and medium-chain triglyceride (MCT), glycerin fatty acid ester, fatty acid acyl ester, and paraffin. Among these oily substances, MCT is preferable. In addition, two or more types of the aforementioned oily substances can be used in combination. When a mixture of an oily substance having a high melting point and an oily substance having a low melting point is used, the ratio between the oily substance with a high melting point and the oily substance with a low melting point is preferably 1:10 to 10:1. In such a case, oily substances having structures that are similar to each other are preferably used. Moreover, the oily substance is preferably a liquid at a room temperature, and the melting point of the oily substance is -40 to 40°C, and in particular, it is preferably -30 to 20°C.

The content of the porous microparticulate powder is 0.1 to 20 parts by weight, preferably 5 to 15 parts by weight, and more preferably 6 to 10 parts by weight, based on 100 parts by weight of the oily substance.

In a method of preparing an oily substance containing a porous microparticulate powder, a porous microparticulate powder can be added to an oily substance, and can be dispersed therein. Examples of the dispersion method that can be used herein include stirring with human hands, and conventionally known methods such as propeller stirring, turbine-type stirring or Homomixer-type stirring. Moreover, the oily substance can also comprise components other than the porous microparticulate powder.

In the present invention, the aqueous solution is not particularly limited, as long as it is water or a liquid product formed by dissolving a hydrophilic substance in water. A plurality of hydrophilic substances can also be dissolved in water. The hydrophilic substance used in the present invention is not particularly limited, as long as it has a functional group, which easily binds to a water molecule such as a hydroxyl group, a carboxyl group, an amino group, a ketone group or a sulfo group, in a portion of a molecular structure thereof, and is soluble in water. Examples of such a hydrophilic substance include substances used for various intended uses in the field of medicines, quasi drugs, food products, condiments, perfumes, herbal medicines and cosmetic products, such as water-soluble vitamin, organic acid and a salt thereof, sugar, nucleic acid, and other water extracts used in traditional Chinese medicine.

The aqueous solution can comprise components, other than a hydrophilic substance and water. Examples of such an aqueous solution include low-fluidity liquid products with a water content of 15% to 40%, including food extracts such as a blueberry extract, lemon juice, a garlic extract or fresh water clam extract, and pharmaceutical extracts such as a carrot extract or a dong quai extract. Moreover, soft microparticles, which are produced by dissolving a water-soluble polymer such as agar, gelatin, carrageenan, sodium alginate, pullulan, glucomannan, gum Arabic, furcellaran, Eucheuma algae, gellan gum, hydroxypropylmethyl cellulose, hydroxypropyl cellulose, polyvinyl pyrrolidone, polyvinyl alcohol or starch in water, are also included in the aqueous solution for convenience sake.

The content of the aqueous solution is 0.01 to 80 parts by weight, preferably 0.1 to 40 parts by weight, and more preferably 0.2 to 20 parts by weight, based on 100 parts by weight of the oily substance.

The content of water in the aqueous solution can be adjusted, as appropriate, depending on the amount of a hydrophilic substance to be dissolved therein. It is preferably an amount, in which the hydrophilic substance to be dissolved can be dissolved therein.

In the present invention, the state in which the aqueous solution is enclosed in the oily substance is not particularly limited, as long as the aqueous solution is covered with the oily substance and the aqueous solution is present inside of the oily substance. The number of aqueous solutions enclosed in the oily substance can be either one or more.

In the present invention, an example of the method for producing a capsule content, in which an aqueous solution is enclosed in an oily substance containing a porous microparticulate powder, is a method, which comprises adding an aqueous solution to an oily substance containing a porous microparticulate powder, and then stirring the mixture. In such a method, the added aqueous solution can also be enclosed in the oily substance in a state in which the aqueous solution is separated into a plurality of aqueous solutions by stirring and the plurality of aqueous solutions are dispersed in the oily substance.

It is also possible to produce a capsule content comprising a hydrophilic substance dissolved in an aqueous solution, and an incompatible component. Examples of the method for producing such a capsule content include: a method, which comprises adding an incompatible powdery component as well as an aqueous solution, to an oily substance containing a porous microparticulate powder, and then stirring them; and a method, which comprises preparing an oily substance containing a porous microparticulate powder and an incompatible powdery component, then adding an aqueous solution to the prepared oily substance, and then stirring them. The method of preparing an oily substance containing a porous microparticulate powder and an incompatible powdery component is the same as the method of preparing an oily substance containing a porous microparticulate powder.

When such an incompatible component is a hydrophobic component, there can be applied a method, which comprises preparing an oil substance containing a porous microparticulate powder, in which such an incompatible component is dissolved, then adding an aqueous solution to the prepared oily substance, and then stirring them.

In the present invention, the hydrophilic film is not particularly limited, as long as it is a film produced using a hydrophilic substance as a film-forming material. Even if such a film is a hydrophilic film, water contained in the aqueous solution is not allowed to come into contact with the film because the aqueous solution in the capsule content is enclosed in the oily substance, and thus, the softening of the film does not occur.

Examples of the film-forming material include hydrophilic polymers such as gelatin, carrageenan, agar, sodium alginate, pullulan, glucomannan, gum Arabic, furcellaran, Eucheuma algae, gellan gum, hydroxypropylmethyl cellulose, hydroxypropyl cellulose, polyvinyl pyrrolidone, polyvinyl alcohol or starch. Among others, gelatin is preferable.

Upon production of the hydrophilic film, a plasticizer such as glycerin, a pH adjuster such as sodium phosphate, a chelating agent such as trisodium citrate or sodium metaphosphate, a gelation accelerator such as calcium lactate or potassium chloride, a surfactant such as polyglycerin fatty acid ester or lecithin, a sweetener, a perfume, an antiseptic, a coloring agent, etc. can be added to the film-forming material.

The capsule formulation of the present invention is produced by encapsulating a capsule content with a hydrophilic film according to a known method for producing a capsule formulation, such as a plate method, a rotary die method or a seamless method. The major axis of the capsule formulation is not particularly limited, and it is 1 to 50 mm, and preferably, 5 to 20 mm.

Hereinafter, the present invention will be described in detail. However, these examples are not intended to limit the scope of the present invention.

### Examples

### [Example 1]

### [Preservation test of agar granules]

In order to confirm whether or not the capsule formulation of the present invention can stably retain an aqueous solution in a capsule thereof, a capsule formulation was produced using agar granules, and the condition of agar after preservation of the capsule formulation was then examined.

### (Production of agar solution used for agar granules)

1 Part by weight of agar and 0.01 part by weight of Red No. 102 were added to 100 parts by weight of water, and they were then dissolved in the water under heating at 95°C to obtain an agar solution used for agar granules.

### (Production of agar granules)

Using a seamless soft capsule production apparatus, the above described agar solution used for agar granules was dropped, so as to produce soft agar granules having a diameter of approximately 1 mm.

### (Production of capsule film-forming solution)

30 Parts by weight of glycerin and 100 parts by weight of gelatin were added to 90 parts by weight of water, so that they were allowed to absorb water and were swollen. Thereafter, they were dissolved in the water under heating at 80°C to obtain a capsule film-forming solution.

### (Production of capsule content)

10 Parts by weight of AEROSIL RX200 (manufactured by NIPPON AEROSIL CO., LTD.) was added to 100 parts by weight of MCT (COCONARD MT, manufactured by Kao Corporation), and the obtained mixture was then stirred using a stirrer. Thereafter, 5 parts by weight of the above described agar granules were added to the reaction mixture, and the thus obtained mixture was further blended to obtain a capsule content.

### (Production of agar granule-containing soft capsule)

Using a rotary die type soft capsule production apparatus, the capsule content was encapsulated with the capsule film-forming solution, and it was then dried at 30°C for 20 hours to produce an agar granule-containing soft capsule. A single agar granule-containing soft capsule, as obtained above, comprised approximately 10 agar granules therein. The agar granules were soft even after completion of the drying step in the capsule production, and retained water.

### (Evaluation after preservation)

The obtained agar granule-containing soft capsules were preserved at 40°C for 6 months. A photograph of the agar granule-containing soft capsule after completion of the preservation is shown in Figure 1. The point indicated with the arrow in the capsule content is an agar granule. When agar granules enclosed in the agar granule-containing soft capsules after completion of the preservation were observed, the agar granules were soft, at the same level as the agar granules enclosed in the agar granule-containing soft capsules immediately after the production of the capsules, and also, the agar granules after completion of the preservation retained water at the same level of the agar granules enclosed in the agar granule-containing soft capsules immediately after the production of the capsules. Moreover, the softening of the film did not occur.

### [Comparative Example 1]

### (Production of agar granule-containing capsule by prior art technique)

An agar granule-containing soft capsule was produced under the same conditions as those of Example 1, with the exception that beeswax was used instead of AEROSIL RX200 in the same amount as that described in Example 1, in the capsule content of Example 1. A single agar granule-containing soft capsule, as obtained above, comprised approximately 10 agar granules therein. The agar granules were soft and retained water immediately after the production thereof.

### (Evaluation after preservation)

The obtained agar granule-containing soft capsules were preserved at 40°C for 6 months. After completion of the preservation, the films of the soft capsules were softened, and the capsules were fused with one another. In addition, water was evaporated from the agar granules, and only the residue was observed.

As described above, it has become clear that an aqueous solution, which could be neither encapsulated in, nor retained by the conventional capsule formulations, can be retained over a long period of time by using the capsule formulation of the present invention, and also that even if a hydrophilic film is used, the films of the capsules are not softened because of the aqueous solution contained in the capsule content.

### [Example 2]

### [Water dispersion test 1 of MCT-AEROSIL liquid mixture]

7 Parts by weight of AEROSIL 380 (manufactured by NIPPON AEROSIL CO., LTD.) was added to 83 parts by weight of MCT (COCONARD MT, manufactured by Kao Corporation), and the obtained mixture was then stirred at 2000 rpm for 5 minutes, using a Homomixer. 10 Parts by weight of colored water was added to the obtained liquid mixture, and the thus obtained mixture was then stirred again at 2000 rpm for 5 minutes to obtain a dispersion.

The obtained dispersion was placed in a centrifuge tube, and was then centrifuged at 2000 rpm for 5 minutes, using a centrifugal separator. After completion of the centrifugation, the colored water was not separated from MCT, and the reaction solution was a uniform dispersion.

### [Example 3]

### [Water dispersion test 2 of MCT-AEROSIL liquid mixture]

5 Parts by weight of AEROSIL 200 (manufactured by NIPPON AEROSIL CO., LTD.) was added to 85 parts by weight of MCT (COCONARD MT, manufactured by Kao Corporation), and the obtained mixture was then stirred at 2000 rpm for 5 minutes, using a Homomixer. 10 Parts by weight of colored water was added to the obtained liquid mixture, and the thus obtained mixture was then stirred again at 2000 rpm for 5 minutes to obtain a dispersion.

The obtained dispersion was placed in a centrifuge tube, and was then centrifuged at 2000 rpm for 5 minutes, using a centrifugal separator. After completion of the centrifugation, the colored water was not separated from MCT, and the reaction solution was a uniform dispersion.

Accordingly, if an aqueous solution was enclosed in an MCT-AEROSIL liquid mixture, the aqueous solution can be maintained in a state in which it was enclosed in the MCT-AEROSIL liquid mixture. When it is used as a capsule content, it becomes possible to prevent evaporation of water from the aqueous solution or to prevent the softening of the film.

### [Example 4]

### [Preservation stability test of cyanocobalamin]

A capsule formation comprising, in a single capsule, an aqueous solution prepared by dissolving cyanocobalamin as a hydrophilic vitamin in water, and thiamine and pyridoxine, which deteriorate such cobalamin, was produced, and degradation of the cyanocobalamin was examined. It is to be noted that the phrase "degradation of the cyanocobalamin" means a reduction in the amount of cyanocobalamin due to decomposition or the like.

### (Production of capsule content in which cyanocobalamin aqueous solution is enclosed)

1654.68 parts by weight of MCT (COCONARD MT, manufactured by Kao Corporation) was heated to 60°C, and 116.1 parts by weight of AEROSIL RX200 (manufactured by NIPPON AEROSIL CO., LTD.) was then added to the reaction mixture. The thus obtained mixture was stirred using a stirrer, while cooling with water. Thereafter, 6 parts by weight of 1% cyanocobalamin aqueous solution, 109.16 parts by weight of fursultiamine hydrochloride, 20 parts by weight of pyridoxine hydrochloride, and 800 parts by weight of sodium chondroitin sulfate ester were added to the reaction mixture, and the thus obtained mixture was further stirred and mixed. Thereafter, the reaction mixture was crushed using a colloid mill, and was then subjected to vacuum defoaming, so as to obtain a capsule content, in which a cyanocobalamin aqueous solution was enclosed in AEROSIL-containing MCT.

### (Production of cyanocobalamin-containing capsule)

Employing a rotary die type soft capsule production apparatus, a soft capsule was produced using the capsule film-forming solution described in Example 1 and the above described capsule content in which a cyanocobalamin aqueous solution was enclosed.

### (Evaluation after preservation)

The obtained cyanocobalamin-containing soft capsule formulations were preserved at 40°C for 1 month. After completion of the preservation, the aqueous solution was maintained in the soft capsules in the same amount as that immediately after production of the capsules, and the softening of the film did not occur. Moreover, the amount of cyanocobalamin immediately after production of the soft capsules and the amount of cyanocobalamin after preservation for 1 month were measured according to a high performance liquid chromatography method. The results are shown in Table 1. The amount of cyanocobalamin in Table 1 indicates the amount of cyanocobalamin (%) immediately after production and the amount of cyanocobalamin (%) after preservation for 1 month, when the amount of cyanocobalamin added is defined as 100.

**[Table 1]**

| Specimen | Amount of cyanocobalamin (%) |
|---|---|
| Immediately after production | 99.8 |
| After preservation at 40°C for 1 month | 94.0 |

### [Comparative Example 2]

### (Production of cyanocobalamin-containing capsule by prior art technique)

A cyanocobalamin-containing capsule formulation was produced by the same method as that of Example 4, with the exception that POEM S-100 (manufactured by Riken Vitamin Co., Ltd.) was used instead of AEROSIL RX200 in the same amount as that described in Example 4, in the cyanocobalamin-containing capsule content of Example 4.

### (Evaluation after preservation)

The amount of cyanocobalamin immediately after production of soft capsules and the amount of cyanocobalamin after preservation for 1 month were measured in the same manner as that of Example 4. The results are shown in Table 2. The amount of cyanocobalamin in Table 2 indicates the amount of cyanocobalamin (%) immediately after production and the amount of cyanocobalamin (%) after preservation for 1 month, when the amount of cyanocobalamin added is defined as 100.

**[Table 2]**

| Specimen | Amount of cyanocobalamin (%) |
|---|---|
| Immediately after production | 94.9 |
| After preservation at 40°C for 1 month | 68.9 |

From the results given above, it has become clear that an aqueous solution can be retained over a long period of time by using the capsule formulation of the present invention, and thus that, by dissolving a hydrophilic substance in the aqueous solution, it becomes possible to stably retain a hydrophilic substance for a long period of time and also to prevent the softening of the film. Moreover, it has also become clear that if the capsule formulation of the present invention is used, degradation of a hydrophilic substance can be prevented by dissolving the hydrophilic substance in the aqueous solution, even if the hydrophilic substance and an incompatible component is mixed into the capsule content.

### Industrial Applicability

The capsule formulation of the present invention can be used as a capsule formulation capable of stably retaining a hydrophilic substance for a long period of time in the field of medicines, quasi drugs, food products, etc.

## Claims

1. A capsule formulation, produced by encapsulating with a hydrophilic film, a capsule content formed by enclosing soft microparticles, which are produced by dissolving a water-soluble polymer in water, in an oily substance containing a porous microparticulate powder.

2. The capsule formulation according to claim 1, which comprises 0.1 to 20 parts by weight of porous microparticulate powder based on 100 parts by weight of oily substance.

3. The capsule formulation according to claim 1 or 2, which is a soft capsule formulation.

4. The capsule formulation according to any one of claims 1 to 3, wherein the porous microparticulate powder is a fumed silica.

## Patentansprüche

1. Kapselformulierung, erzeugt durch Verkapselung mit einem hydrophilen Film, eines Kapselinhalts, der durch Umschließen von weichen Mikropartikeln gebildet wird, die durch Lösen eines wasserlöslichen Polymers in Wasser erzeugt werden, in einer öligen Substanz, die ein poröses mikropartikuläres Pulver enthält.

2. Kapselformulierung nach Anspruch 1, die 0,1 bis 20 Gewichtsteile poröses mikropartikuläres Pulver umfasst, basierend auf 100 Gewichtsteile öliger Substanz.

3. Kapselformulierung nach Anspruch 1 oder 2, die eine weiches Kapselformulierung ist.

4. Kapselformulierung nach einem der Ansprüche 1 bis 3, wobei das poröse mikropartikuläre Pulver ein pyrogenes Siliciumdioxid ist.

## Revendications

1. Formulation de capsule, produite par encapsulation avec un film hydrophile, un contenu de capsule formé en enserrant des microparticules molles, qui sont produites en dissolvant un polymère soluble dans l'eau dans de l'eau, dans une substance huileuse contenant une poudre microparticulaire poreuse.

2. Formulation de capsule selon la revendication 1, qui comprend 0,1 à 20 parties en poids de poudre microparticulaire poreuse sur la base de 100 parties en poids de substance huileuse.

3. Formulation de capsule selon la revendication 1 ou 2, qui est une formulation de capsule molle.

4. Formulation de capsule selon l'une quelconque des revendications 1 à 3, dans laquelle la poudre microparticulaire poreuse est une silice fumée.
